(19) Europäisches Patentamt
European Patent Office
Office européen des brevets

(11) **EP 3 796 838 B1**

(12) **FASCICULE DE BREVET EUROPEEN**

(45) Date de publication et mention de la délivrance du brevet:
**20.07.2022 Bulletin 2022/29**

(21) Numéro de dépôt: **19728496.1**

(22) Date de dépôt: **03.05.2019**

(51) Classification Internationale des Brevets (IPC):
*A61B 5/145* (2006.01)    *A61B 5/1495* (2006.01)
*A61B 5/00* (2006.01)    *G16H 20/17* (2018.01)

(52) Classification Coopérative des Brevets (CPC):
**A61B 5/14532; A61B 5/1495; A61B 5/4839; G16H 20/17; G16H 50/30**

(86) Numéro de dépôt international:
**PCT/FR2019/051025**

(87) Numéro de publication internationale:
**WO 2019/224446 (28.11.2019 Gazette 2019/48)**

(54) **SYSTÈME AUTOMATISÉ DE CONTRÔLE DE LA GLYCÉMIE D'UN PATIENT**

AUTOMATISIERTES SYSTEM ZUR ÜBERWACHUNG DES BLUTZUCKERS EINES PATIENTEN

AUTOMATED SYSTEM FOR MONITORING A PATIENT'S BLOOD SUGAR

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **22.05.2018 FR 1800492**

(43) Date de publication de la demande:
**31.03.2021 Bulletin 2021/13**

(73) Titulaire: **Commissariat à l'Energie Atomique et aux Energies Alternatives
75015 Paris (FR)**

(72) Inventeurs:
• **BLANC, Romain**
  **38000 GRENOBLE (FR)**
• **DORON, Eléonore-Maeva**
  **38054 GRENOBLE CEDEX 09 (FR)**
• **ROMERO UGALDE, Hector-Manuel**
  **38000 GRENOBLE (FR)**

(74) Mandataire: **Cabinet Beaumont
4, Place Robert Schuman
B.P. 1529
38025 Grenoble Cedex 1 (FR)**

(56) Documents cités:
**US-A1- 2014 276 556    US-A1- 2015 031 053**

• RITA S. PATARRÃO ET AL: "Assessment of methods and indexes of insulin sensitivity", REVISTA PORTUGUESA DE ENDOCRINOLOGIA, DIABETES E METABOLISMO, vol. 9, no. 1, 1 janvier 2014 (2014-01-01), pages 65-73, XP055520898, ISSN: 1646-3439, DOI: 10.1016/j.rpedm.2013.10.004
• XIN-LONG ET AL: "Insulin resistance following thermal injury: An animal study", BURNS, BUTTERWORTH HEINEMANN, GB, vol. 33, no. 4, 10 mai 2007 (2007-05-10), pages 480-483, XP022068983, ISSN: 0305-4179, DOI: 10.1016/J.BURNS.2006.08.017

**Description**

La présente demande de la brevet revendique la priorité de la demande de brevet français FR18/00492.
Domaine

La présente demande concerne le domaine des systèmes automatisés de contrôle de glycémie, et vise plus particulièrement, dans un tel système, la détermination d'un coefficient représentatif de la sensibilité à l'insuline du patient.

Le document US2014/276556 décrit un exemple de système automatisé de contrôle de glycémie.

Exposé de l'art antérieur

On a déjà proposé, par exemple dans la demande de brevet français N°1658881 (B15018/DD16959) déposée le 21 septembre 2016, dans la demande de brevet français N°1658882 (B15267/ DD17175) déposée le 21 septembre 2016, et dans la demande de brevet français N°1756960 (B15860/DD18480) déposée le 21 juillet 2017, des systèmes automatisés de régulation de glycémie, aussi appelés pancréas artificiels, permettant de réguler automatiquement les apports en insuline d'un patient diabétique à partir de son historique de glycémie, de son historique de prise de repas, et de son historique d'injection d'insuline.

Les systèmes de régulation décrits dans les demandes de brevet susmentionnées sont des systèmes de type MPC (de l'anglais "Model-based Prédictive Control"), aussi appelés systèmes à commande prédictive, dans lesquels la régulation de la dose d'insuline administrée tient compte d'une prédiction de l'évolution future de la glycémie du patient, réalisée à partir d'un modèle physiologique décrivant l'assimilation de l'insuline par le corps du patient et son impact sur la glycémie du patient.

Plus généralement, de nombreux systèmes automatisés de contrôle de glycémie tiennent compte de l'historique de glycémie, de l'historique de prise de repas, et de l'historique d'injection d'insuline d'un patient pour déterminer des doses d'insuline à administrer au patient en vue de maintenir sa glycémie dans une plage souhaitée.

Dans les systèmes automatisés de contrôle de glycémie, un paramètre qui joue un rôle essentiel dans la détermination des doses d'insuline à administrer au patient est le coefficient de sensibilité à l'insuline du patient, aussi appelée coefficient de sensibilité de compensation, ou ratio de compensation, c'est-à-dire la quantité d'insuline nécessaire pour faire baisser la glycémie de un gramme par litre (en UI/g/l - où UI désigne une unité internationale d'insuline, soit l'équivalent biologique d'environ 0,0347 mg d'insuline humaine).

Un problème qui se pose est que le coefficient de sensibilité à l'insuline peut varier de façon significative d'un patient à un autre, ou, chez un même patient, en fonction des conditions dans lesquelles se trouve le patient (niveau glycémique, activité physique, rythme nycthéméral, stress, etc.).

En pratique, les systèmes automatisés de contrôle de glycémie connus se basent sur un coefficient de sensibilité à l'insuline fixe, par exemple fourni par le diabétologue du patient. Il n'est donc pas tenu compte des variations à court terme du coefficient. Il en résulte que les quantités d'insuline injectées au patient sont parfois inadaptées, entrainant un risque d'hyperglycémie ou d'hypoglycémie.

Les demandes de brevet US2010/0198520, US2013/0211220 et WO2017/040927 décrivent des exemples de méthodes de détermination du coefficient de sensibilité à l'insuline d'un patient en tenant compte de son historique de glycémie, de son historique d'injection d'insuline et de son historique de prise de repas. Ces méthodes sont toutefois relativement complexes à mettre en oeuvre et nécessitent de disposer d'un historique de données sur une période d'observation passée relativement longue. En particulier, ces méthodes ne permettent pas d'ajuster le coefficient de sensibilité à l'insuline en temps réel en fonction des évolutions de la situation du patient.

Résumé

Ainsi, un mode de réalisation prévoit un système automatisé de contrôle de la glycémie d'un patient, comportant un capteur de glycémie et une unité de traitement et de contrôle, dans lequel l'unité de traitement et de contrôle est configurée pour calculer, à partir d'un premier modèle mathématique $f_{CR}$ spécifique au patient et en tenant compte d'une unique valeur de glycémie $G^r$ mesurée par le capteur, un coefficient CR représentatif de la sensibilité à l'insuline du patient.

Selon un mode de réalisation, le système comporte en outre un dispositif d'injection d'insuline, et l'unité de traitement et de contrôle est configurée pour commander le dispositif d'injection d'insuline en tenant compte du coefficient CR.

Selon un mode de réalisation, l'unité de traitement et de contrôle est configurée pour prédire, à partir d'un deuxième modèle mathématique, l'évolution future de la glycémie du patient sur une période de prédiction, et à commander le dispositif d'injection d'insuline en tenant compte de cette prédiction.

Selon un mode de réalisation, le premier modèle mathématique est une fonction d'équation

$$CR = f_{CR}(G^r) = a \times G^{rb} + c$$

où a, b et c sont des paramètres spécifiques au patient.

Selon un mode de réalisation, l'unité de traitement et de contrôle est configurée pour mettre en oeuvre une étape de calibration automatique du premier modèle $f_{CR}$ en tenant compte d'un historique de glycémie mesurée par le capteur, d'un historique d'insuline injectée au patient, et d'un historique d'ingestion de glucose par le patient sur une période d'observation passée.

Selon un mode de réalisation, l'unité de traitement et de contrôle est configurée pour, lors de l'étape de calibration automatique, mesurer une pluralité de valeurs du coefficient de sensibilité à l'insuline réel $CR^r$ du patient lors d'une pluralité d'événements de mesure compris dans la période d'observation passée.

Selon un mode de réalisation, chaque évènement de mesure correspond à une plage temporelle continue allant d'un instant initial $t_{init}$ à un instant final $t_{final}$, répondant aux critères suivants :

- l'instant $t_{init}$ se situe dans une phase d'hyperglycémie, c'est-à-dire une phase dans laquelle la glycémie du patient est supérieure à un seuil prédéterminé ;
- un bolus de correction, c'est-à-dire une dose d'insuline, a été administré au patient après le début de la phase d'hyperglycémie et avant l'instant $t_{init}$, en vue de limiter la durée de la phase d'hyperglycémie ;
- la glycémie du patient décroit de façon continue entre l'instant initial $t_{init}$ et l'instant final $t_{final}$ ;
- aucune ingestion de glucose par le patient n'a été effectuée entre l'instant $t_{init}$ - Tj et l'instant $t_{final}$, où $T_j$ est une durée de jeun prédéterminée.

Selon un mode de réalisation, l'unité de traitement et de contrôle est configurée pour, lors de l'étape de calibration automatique, déterminer le premier modèle mathématique $f_{CR}$ par régression à partir de ladite pluralité de valeurs du coefficient de sensibilité à l'insuline réel $CR^r$.

Selon un mode de réalisation, l'unité de traitement et de contrôle est configurée pour, lors d'une phase initiale $T_{pop}$ d'utilisation du système précédant l'étape de calibration automatique du premier modèle, utiliser un modèle mathématique générique $f_{CR-pop}$ non personnalisé pour calculer le coefficient CR.

Selon un mode de réalisation, l'unité de traitement et de contrôle est configurée pour, lors d'une phase intermédiaire $T_{hyb}$ d'utilisation du système, postérieure à la phase initiale $T_{pop}$ et antérieure à l'étape de calibration automatique du premier modèle, utiliser un modèle mathématique partiellement personnalisé $f_{CR-hyb}$ pour calculer le coefficient CR.

Selon un mode de réalisation, le modèle mathématique partiellement personnalisé est défini par l'équation :

$$f_{CR-hyb}(G^r) = k \times f_{CR-pop}(G^r)$$

où k est un coefficient spécifique au patient défini selon la formule suivante :

$$k = \frac{TDD_{moy}}{a1 \times BW}$$

où BW désigne le poids du patient, $TDD_{moy}$ désigne la dose d'insuline journalière moyenne injectée au patient sur la période $T_{pop}$, et a1 est un coefficient constant compris entre 0,5 et 0,9.

Selon un mode de réalisation, l'unité de traitement et de contrôle est configurée pour, après l'étape de calibration automatique du premier modèle $f_{CR}$, mettre en oeuvre une pluralité d'étapes successives de re-calibration du premier modèle pour tenir compte de nouvelles données de glycémie mesurée par le capteur, d'insuline injectée au patient, et d'ingestion de glucose par le patient.

Brève description des dessins

Ces caractéristiques et avantages, ainsi que d'autres, seront exposés en détail dans la description suivante de modes de réalisation particuliers faite à titre non limitatif en relation avec les figures jointes parmi lesquelles :

la figure 1 représente de façon schématique, sous forme de blocs, un exemple d'un système automatisé de régulation de la glycémie d'un patient selon un mode de réalisation ;
la figure 2 est une représentation simplifiée d'un modèle physiologique utilisé dans le système de la figure 1 pour prédire l'évolution future de la glycémie du patient ;

la figure 3 est un diagramme illustrant un exemple d'un procédé automatisé de régulation de glycémie pouvant être mis en oeuvre par le système de la figure 1 ;

les figures 4A et 4B sont des diagrammes représentant l'évolution du coefficient de sensibilité à l'insuline d'un patient en fonction de sa glycémie ;

la figure 5 est une représentation simplifiée d'un modèle mathématique utilisé dans le système de la figure 1 pour déterminer le coefficient de sensibilité à l'insuline du patient ;

la figure 6 est un diagramme illustrant un exemple d'un procédé pouvant être mis en oeuvre par le système de la figure 1 pour mettre à jour le modèle mathématique de la figure 5 ;

la figure 7 est une représentation simplifiée d'un autre exemple d'un modèle mathématique utilisé dans le système de la figure 1 pour déterminer le coefficient de sensibilité à l'insuline du patient ;

la figure 8 est une représentation simplifiée d'un autre exemple d'un modèle mathématique utilisé dans le système de la figure 1 pour déterminer le coefficient de sensibilité à l'insuline du patient ; et

la figure 9 est un diagramme illustrant un exemple d'un procédé de contrôle de la glycémie d'un patient pouvant être mis en oeuvre par le système de la figure 1.

## Description détaillée

De mêmes éléments ont été désignés par de mêmes références dans les différentes figures et, de plus, les diverses figures ne sont pas tracées à l'échelle. Par souci de clarté, seuls les éléments utiles à la compréhension des modes de réalisation décrits ont été représentés et sont détaillés. En particulier, la réalisation matérielle de l'unité de traitement et de contrôle des systèmes décrits n'a pas été détaillée, la réalisation d'une telle unité de traitement et de contrôle étant à la portée de l'homme du métier à partir des indications fonctionnelles de la présente description. En outre, le dispositif de mesure de glycémie et le dispositif d'injection d'insuline des systèmes décrits n'ont pas été détaillés, les modes de réalisation décrits étant compatibles avec tous ou la plupart des dispositifs de mesure de glycémie et d'injection d'insuline connus. Sauf précision contraire, les expressions "approximativement", "sensiblement", et "de l'ordre de" signifient à 10 % près, de préférence à 5 % près.

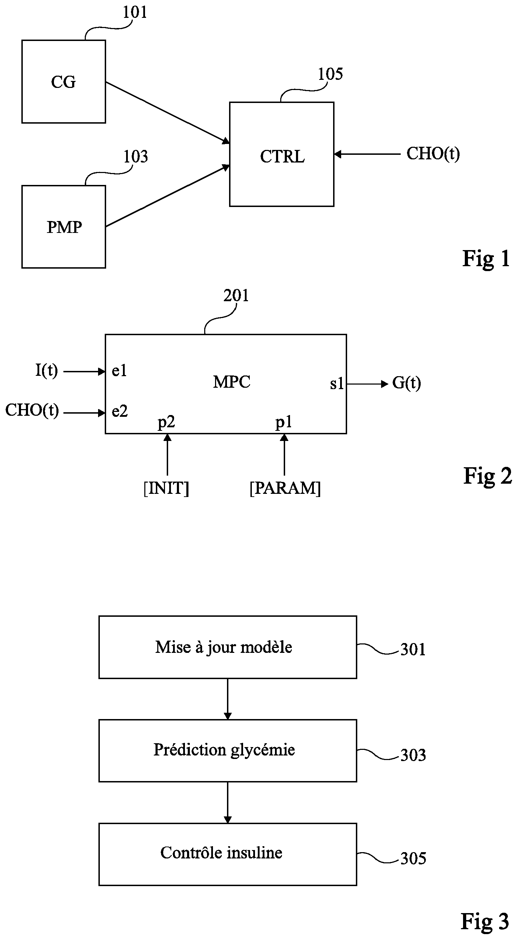

La figure 1 représente de façon schématique, sous forme de blocs, un exemple d'un mode de réalisation d'un système automatisé de régulation de la glycémie d'un patient.

Le système de la figure 1 comprend un capteur 101 (CG) adapté à mesurer la glycémie du patient. En fonctionnement normal, le capteur 101 peut être positionné à demeure sur ou dans le corps du patient, par exemple à hauteur de son abdomen. Le capteur 101 est par exemple un capteur de type CGM (de l'anglais "Continuous Glucose Monitoring" - surveillance continue de glycémie), c'est-à-dire un capteur adapté à mesurer en continu, par exemple de façon périodique (par exemple au moins une fois toutes les cinq minutes) la glycémie du patient. Le capteur 101 est par exemple un capteur de glycémie en sous-cutané.

Le système de la figure 1 comprend en outre un dispositif d'injection d'insuline 103 (PMP), par exemple un dispositif d'injection en sous-cutané. Le dispositif 103 est par exemple un dispositif d'injection automatique de type pompe à insuline, comportant un réservoir d'insuline relié à une aiguille d'injection implantée sous la peau du patient, la pompe pouvant être commandée électriquement pour injecter automatiquement des doses d'insuline déterminées à des instants déterminés. En fonctionnement normal, le dispositif d'injection 103 peut être positionné à demeure dans ou sur le corps du patient, par exemple au niveau de son abdomen.

Le système de la figure 1 comprend en outre une unité de traitement et de contrôle 105 (CTRL) reliée d'une part au capteur de glycémie 101, par exemple par liaison filaire ou par liaison radio (sans fil), et d'autre part au dispositif d'injection 103, par exemple par liaison filaire ou radio. En fonctionnement, l'unité de traitement et de contrôle 105 est adaptée à recevoir les données de glycémie du patient mesurées par le capteur 101, et à commander électriquement le dispositif 103 pour injecter au patient des doses d'insuline déterminées à des instants déterminés. Dans cet exemple, l'unité de traitement et de contrôle 105 est en outre adaptée à recevoir, par l'intermédiaire d'une interface utilisateur non détaillée, des données CHO(t) représentatives de l'évolution, en fonction du temps, de la quantité de glucose ingérée par le patient.

L'unité de traitement et de contrôle 105 est adaptée à déterminer les doses d'insuline à injecter au patient en tenant compte notamment de l'historique de glycémie mesurée par le capteur 101, de l'historique d'insuline injectée par le dispositif 103, et de l'historique d'ingestion de glucose par le patient. Pour cela, l'unité de traitement et de contrôle 105 comprend un circuit de calcul numérique (non détaillé), comprenant par exemple un microprocesseur. L'unité de traitement et de contrôle 105 est par exemple un dispositif mobile porté par le patient tout au long de la journée et/ou de la nuit, par exemple un dispositif de type smartphone configuré pour mettre en oeuvre un procédé de régulation du type décrit ci-après.

Dans l'exemple de la figure 1, l'unité de traitement et de contrôle 105 est adaptée à déterminer la quantité d'insuline à administrer au patient en tenant compte d'une prédiction de l'évolution future de sa glycémie en fonction du temps. Plus particulièrement, l'unité de traitement et de contrôle 105 est adaptée, à partir de l'historique d'insuline injectée et de l'historique de glucose ingéré, et en se basant sur un modèle mathématique, par exemple un modèle physiologique décrivant l'assimilation de l'insuline par le corps du patient et son impact sur la glycémie, à déterminer une courbe représentative de l'évolution attendue de la glycémie du patient en fonction du temps, sur une période à venir appelée période de prédiction ou horizon de prédiction, par exemple une période de 1 à 10 heures. En tenant compte de cette courbe et d'un coefficient représentatif de la sensibilité à l'insuline du patient, l'unité de traitement et de contrôle 105 détermine les doses d'insuline qu'il convient d'injecter au patient pendant la période de prédiction à venir, pour que la glycémie réelle (par opposition à la glycémie estimée à partir du modèle mathématique) du patient reste dans des limites acceptables, et en particulier pour limiter les risques d'hyperglycémie ou d'hypoglycémie.

La figure 2 est une représentation simplifiée d'un modèle mathématique 201 (MPC) utilisé dans le système de la figure 1 pour prédire l'évolution future de la glycémie du patient. Sur la figure 2, le modèle est représenté sous la forme d'un bloc de traitement comportant :

une entrée e1 sur laquelle est appliqué un signal I(t) représentatif de l'évolution, en fonction du temps t, de la quantité d'insuline injectée au patient ;
une entrée e2 sur laquelle est appliqué un signal CHO(t) représentatif de l'évolution, en fonction du temps t, de la quantité de glucose ingérée par le patient ; et
une sortie s1 fournissant un signal G(t) représentatif de l'évolution, en fonction du temps t, de la glycémie estimée du patient.

Le modèle mathématique 201 est par exemple un modèle physiologique. A titre d'exemple, le modèle 201 est un modèle physiologique compartimentai comportant, outre les variables d'entrée I(t) et CHO(t) et la variable de sortie G(t), une pluralité de variables d'états correspondant à des variables physiologiques du patient, évoluant en fonction du temps. L'évolution temporelle des variables d'état et de la variable de sortie G(t) est régie par un système d'équations différentielles comportant une pluralité de paramètres représentés sur la figure 2 par un vecteur [PARAM] appliqué sur une entrée p1 du bloc 201. La réponse du modèle physiologique est en outre conditionnée par les états initiaux ou valeurs initiales affectés aux variables d'état, représentés sur la figure 2 par un vecteur [INIT] appliqué sur une entrée p2 du bloc 201.

A titre d'exemple, le modèle physiologique 201 utilisé dans le système de la figure 1 est le modèle dit de Hovorka, décrit dans l'article intitulé "Nonlinear model prédictive control of glucose concentration in subjects with type 1 diabetes" de Roman Hovorka et al. (Physiol Meas. 2004;25:905-920), et dans l'article intitulé "Partitioning glucose distribution/transport, disposai, and endogenous production during IVGTT", de Roman Hovorka et al. (Am J Physiol Endocrinol Metab 282: E992-E1007, 2002). Plus généralement, tout autre modèle physiologique décrivant l'assimilation de l'insuline par le corps d'un patient et son effet sur la glycémie du patient peut être utilisé.

Parmi les paramètres du vecteur [PARAM], certains peuvent être considérés comme constants pour un patient donné. D'autres paramètres, appelés ci-après paramètres temps-dépendants, sont en revanche susceptibles d'évoluer dans le temps. Du fait de cette variabilité de certains paramètres du système, il est en pratique nécessaire de ré-étalonner ou re-calibrer régulièrement le modèle en cours d'utilisation, par exemple toutes les 1 à 20 minutes, par exemple toutes les 5 minutes, pour s'assurer que les prédictions du modèle restent pertinentes. Cette mise à jour du modèle, aussi appelée personnalisation du modèle, doit de préférence pouvoir être réalisée de façon automatique par le système de la figure 1, c'est-à-dire sans qu'il soit nécessaire de mesurer physiquement les paramètres temps-dépendants du système sur le patient puis de les transmettre à l'unité de traitement et de contrôle 105.

La figure 3 est un diagramme illustrant un exemple d'un procédé automatisé de régulation de glycémie pouvant être mis en oeuvre par le système de la figure 1.

Ce procédé comprend une étape 301 de re-calibration ou mise à jour du modèle, pouvant par exemple être répétée à intervalles réguliers, par exemple toutes les 1 à 20 minutes. Lors de cette étape, l'unité de traitement et de contrôle 105 met en oeuvre un procédé de ré-estimation des paramètres temps-dépendants du modèle en tenant compte des données d'insuline effectivement injectée par le dispositif 103 et des données de glycémie réelle mesurée par le capteur 101 pendant une période d'observation passée de durée $\Delta T$, par exemple une période de 1 à 10 heures précédant l'étape de calibration. Plus particulièrement, lors de l'étape de calibration, l'unité de traitement et de contrôle 105 simule le comportement du patient sur la période d'observation passée à partir du modèle physiologique (en tenant compte des éventuelles ingestions de glucose et injections d'insuline pendant cette période), et compare la courbe de glycémie estimée par le modèle à la courbe de glycémie réelle mesurée par le capteur pendant cette même période. L'unité de traitement et de contrôle 105 recherche alors, pour les paramètres temps-dépendants du modèle, un jeu de valeurs conduisant à minimiser une grandeur représentative de l'erreur entre la courbe de glycémie estimée par le modèle et la courbe de glycémie réelle pendant la période d'observation. A titre d'exemple, l'unité de traitement et de contrôle recherche un jeu de paramètres conduisant à minimiser un indicateur m représentatif de l'aire entre la courbe de glycémie estimée par le modèle et la courbe de glycémie réelle pendant la période d'observation, aussi appelé écart quadratique moyen entre la glycémie estimée et la glycémie réelle, par exemple défini comme suit :

$$m = \frac{1}{\Delta T}\sum_{t=t_0-\Delta T}^{t_0}|G^r(t) - G(t)|^2 \qquad\qquad (\mathrm{eq1})$$

où t est une variable temps discrétisée, $t_0$-$\Delta T$ correspond à l'instant de début de la phase d'observation passé, $t_0$ correspond à l'instant de fin de la phase d'observation passée (correspondant par exemple à l'instant de début de l'étape de calibration du modèle), $G^r$ est la courbe d'évolution temporelle de la glycémie réelle mesurée par le capteur 101 pendant la période [$t_0$-$\Delta T$, $t_0$], et G est la courbe de glycémie estimée à partir du modèle pendant la période [$t_0$-$\Delta T$, $t_0$]. A titre de variante, pour le calcul de l'écart quadratique moyen, la variable $\Delta T$ peut être remplacée par le nombre de mesures réalisées pendant la période d'observation passée. L'algorithme de recherche de paramètres optimaux utilisé lors de cette étape n'est pas détaillé dans la présente demande, les modes de réalisation décrits étant compatibles avec les algorithmes usuels utilisés dans des domaines variés pour résoudre des problèmes d'optimisation de paramètres par minimisation d'une fonction de coût.

On notera que lors de l'étape 301, outre les paramètres temps-dépendants du modèle, l'unité de traitement et de contrôle 105 définit un vecteur [INIT] d'états initiaux (états à l'instant $t_0$-$\Delta T$) des variables d'état du modèle, pour pouvoir simuler le comportement du patient à partir du modèle. Pour définir les états initiaux des variables d'état du modèle, une première possibilité consiste à faire l'hypothèse que, dans la période précédant la période d'observation [$t_0$-$\Delta T$, $t_0$] sur laquelle est basée la calibration du modèle, le patient se trouvait dans un état stationnaire, avec un débit d'insuline injectée constant, et une prise alimentaire de glucose nulle. Sous cette hypothèse, toutes les dérivées du système d'équations différentielles peuvent être considérées comme nulles à l'instant initial $t_0$-$\Delta T$. Les valeurs à l'instant $t_0$-$\Delta T$ des variables d'état du système peuvent alors être calculées analytiquement. Pour améliorer l'initialisation, une autre possibilité consiste à faire les mêmes hypothèses que précédemment, mais en ajoutant la contrainte que la glycémie estimée à l'instant $t_0$-$\Delta T$ soit égale à la glycémie réelle mesuré par le capteur. Pour améliorer encore l'initialisation, une autre possibilité est de considérer les états initiaux des variables d'état du modèle comme des variables aléatoires, au même titre que les paramètres temps-dépendants du modèle. Les états initiaux des variables d'état sont alors déterminés de la même façon que les paramètres temps-dépendants du modèle, c'est-à-dire que l'unité de traitement et de contrôle 105 recherche un jeu de valeurs d'états initiaux [INIT] conduisant à minimiser une grandeur représentative de l'erreur entre la courbe de glycémie estimée par le modèle et la courbe de glycémie réelle pendant la période d'observation passée.

Le procédé de la figure 3 comprend en outre, après l'étape 301, une étape 303 de prédiction, par l'unité de traitement et de contrôle 105, de l'évolution temporelle de la glycémie du patient sur une période de prédiction à venir [$t_0$, $t_0$+$T_{pred}$] de durée $T_{pred}$, par exemple comprise entre 1 et 10 heures, à partir du modèle physiologique mis à jour à l'étape 301 et en tenant compte de l'historique d'insuline injectée au patient et de l'historique de glucose ingéré par le patient.

Le procédé de la figure 3 comprend de plus, après l'étape 303, une étape 305 de détermination, par l'unité de traitement et de contrôle 105, en tenant compte de la courbe de glycémie future prédite à l'étape 303, des doses d'insuline à injecter au patient pendant la période de prédiction à venir [$t_0$,$t_0$+$T_{pred}$]. A l'issue de cette étape, l'unité de traitement et de contrôle 105 peut programmer le dispositif d'injection 103 pour administrer les doses déterminées pendant la période de prédiction [$t_0$, $t_0$+$T_{pred}$].

Les étapes 303 de prédiction de la glycémie et 305 de détermination des doses futures d'insuline à administrer peuvent par exemple être réitérées à chaque mise à jour du modèle physiologique (c'est-à-dire après chaque itération de l'étape 301), à chaque nouvelle ingestion de glucose signalée par le patient, et/ou à chaque nouvelle administration d'une dose d'insuline par le dispositif d'injection 103.

Selon un aspect d'un mode de réalisation, lors de l'étape 305, l'unité de traitement et de contrôle 105 estime le coefficient de sensibilité à l'insuline CR du patient à partir d'une unique valeur de glycémie mesurée par le capteur 101, en se basant sur un modèle mathématique prédéterminé. Autrement dit, lors de l'étape 305, l'unité de traitement et de contrôle 105 calcule le coefficient de sensibilité à l'insuline CR du patient à partir d'une fonction mathématique prédéterminée $f_{CR}$ telle que CR = $f_{CR}(G^r(t))$, où $G^r(t)$ est la valeur de glycémie réelle du patient mesurée par le capteur 101 à un instant t courant, par exemple l'instant t=$t_0$. Lors de l'étape 305, l'unité de traitement et de contrôle 105 détermine alors les doses futures d'insuline à administrer au patient en tenant compte du coefficient de sensibilité CR ainsi calculé.

Les inventeurs ont montré qu'il existe, pour un patient donné, une corrélation forte entre l'évolution temporelle de la glycémie du patient et l'évolution temporelle du coefficient de sensibilité à l'insuline du patient. Les inventeurs ont notamment montré que l'ajustement en temps réel du coefficient de sensibilité à l'insuline du patient en fonction de sa glycémie instantanée permet de déterminer avec une meilleure précision les doses d'insuline futures à administrer au patient et ainsi limiter les risques d'hyperglycémie ou d'hypoglycémie.

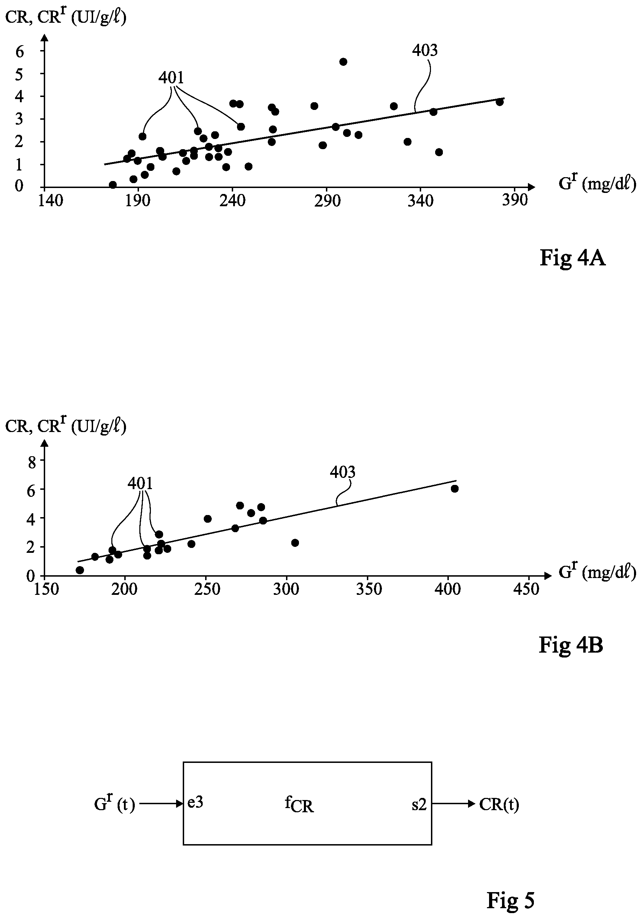

Les figures 4A et 4B sont des diagrammes représentant respectivement, pour deux patients distincts, l'évolution du coefficient de sensibilité à l'insuline du patient (en ordonnée, en UI/g/l) en fonction de sa glycémie (en abscisse, en mg/dl). Chaque diagramme comprend une pluralité de points 401 correspondant chacun à une mesure du coefficient de sensibilité à l'insuline réel $CR^r$ du patient, et à une mesure correspondante (i.e. corrélée temporellement) de la glycémie réelle G$^r$ du patient.

Le coefficient de sensibilité à l'insuline réel CR$^r$ peut être mesuré par toute méthode connue de mesure du coefficient de sensibilité à l'insuline d'un patient, par exemple par des méthodes du type décrit dans les demandes de brevet US2010/0198520, US2013/0211220 et WO2017/040927 susmentionnées.

Dans un mode de réalisation préféré, le coefficient de sensibilité à l'insuline réel CR$^r$ du patient est déterminé à partir de l'historique de glycémie du patient (par exemple mesuré par le capteur 101 dans le système de la figure 1), de son historique de prise de repas, et de son historique d'injection d'insuline, selon la méthode suivante.

A partir de l'historique de données du patient, on identifie des évènements de mesure, c'est-à-dire des plages de temps pendant lesquelles le coefficient de sensibilité à l'insuline est isolé, c'est-à-dire pendant lesquelles on observe une diminution de la glycémie du patient liée à l'administration d'insuline. A titre d'exemple, les évènements sélectionnés sont des plages temporelles continues allant d'un instant initial t$_{init}$ à un instant final t$_{final}$, répondant aux critères suivants :

- l'instant t$_{init}$ se situe dans une phase d'hyperglycémie, c'est-à-dire une phase dans laquelle la glycémie du patient est supérieure à un seuil prédéterminé, par exemple de l'ordre de 1,40 g/l ;
- un bolus de correction, c'est-à-dire une dose d'insuline supplémentaire a été administré au patient après le début de la phase d'hyperglycémie et avant l'instant t$_{init}$, en vue de limiter la durée de la phase d'hyperglycémie ;
- la glycémie du patient décroit de façon continue entre l'instant initial t$_{init}$ et l'instant final t$_{final}$ ;
- aucune ingestion de glucose par le patient n'a été effectuée entre l'instant t$_{init}$ - Tj et l'instant t$_{final}$, où T$_j$ est une durée de jeun prédéterminée, par exemple supérieure ou égale à 1h et de préférence supérieure ou égale à 2h.

A titre d'exemple, l'instant t$_{init}$ correspond au pic de glycémie de la phase d'hyperglycémie. L'instant t$_{final}$ correspond par exemple à un instant de stabilisation ou de remontée glycémique suivant la phase d'hyperglycémie, ou encore à une perturbation telle qu'un repas ou une ingestion de glucose.

Pour chaque évènement identifié, le coefficient de sensibilité à l'insuline réel CR$^r$ du patient est calculé comme suit :

$$CR^r = \Delta I/\Delta G,$$

où $\Delta I$ désigne la quantité d'insuline consommée pendant l'événement et $\Delta G$ désigne la différence entre la glycémie réelle du patient à l'instant t$_{init}$ de début de l'évènement et la glycémie réelle du patient à l'instant t$_{final}$ de fin de l'événement. La quantité d'insuline $\Delta I$ consommée pendant l'événement peut par exemple être calculée en tenant compte des doses d'insuline administrées avant et pendant l'événement, et de la cinétique d'absorption de l'insuline par le corps. A titre d'exemple, la quantité d'insuline $\Delta I$ consommée pendant l'événement correspond à la différence entre la quantité d'insuline embarquée ("insulin on board" en anglais) du patient, c'est-à-dire la quantité d'insuline encore active (c'est-à-dire encore susceptible d'avoir un effet sur la glycémie) à l'instant t$_{init}$ de début de l'événement et la quantité d'insuline embarquée à l'instant t$_{final}$ de fin de l'événement. La détermination de la quantité d'insuline embarquée du patient aux instants t$_{init}$ et t$_{final}$ peut être réalisée par toute méthode connue de détermination de la quantité d'insuline embarquée d'un patient. A titre d'exemple, la détermination de la quantité d'insuline embarquée du patient à un instant t peut être calculée par convolution, sur une période allant d'un instant antérieur à l'instant t jusqu'à l'instant t, d'une courbe représentative de l'évolution, en fonction du temps, de la quantité d'insuline injectée au patient avant l'instant t, et d'une fonction f$_{IOB}$ représentative de la cinétique de consommation de l'insuline par le corps, par exemple la fonction

$$f_{IOB}(t) = \left(1 + \frac{t-1}{\tau}\right) * e^{-\frac{t-1}{\tau}},$$

où t est la variable temps discrétisée et $\tau$ est une constante de temps de durée prédéterminée, par exemple comprise entre 40 et 60 minutes, par exemple de l'ordre de 47 minutes.

Pour chaque événement, la valeur retenue de glycémie réelle du patient est par exemple la valeur de glycémie réelle G$^r$(t$_{init}$) à l'instant t$_{init}$ de début de l'événement.

Pour chaque patient, pour définir une fonction ou un modèle mathématique f$_{CR}$ spécifique au patient, un nombre d'événements Nb$_{ev}$ relativement élevé est d'abord identifié dans les données d'historique du patient, et, pour chaque événement, une valeur de coefficient de sensibilité à l'insuline CR$^r$ et une valeur de glycémie G$^r$ associée sont mesurées. A titre d'exemple, le nombre d'événements Nb$_{ev}$ utilisé pour définir la fonction f$_{CR}$ est compris entre 20 et 100, par exemple entre 30 et 60, par exemple de l'ordre de 40. En pratique, un historique de données de plusieurs semaines à plusieurs mois peut être nécessaire pour obtenir le nombre Nb$_{ev}$ de mesures souhaité. La fonction f$_{CR}$ est ensuite déterminée par régression à partir des Nb$_{ev}$ points de mesure spécifiques au patient (les points 401 des figures 4A et 4B). A titre d'exemple, pour chaque patient, la fonction $f_{CR}$ est obtenue par régression à partir des $Nb_{ev}$ points de mesure 401 obtenus pour le patient. Pour chaque patient, la fonction $f_{CR}$ est alors une fonction d'équation :

$$f_{CR}(G^r) = a * G^{r^b} + c$$

où a, b et c sont des paramètres spécifiques au patient, le paramètre b correspondant à l'ordre du modèle. A titre d'exemple, le paramètre b est fixé égal à 1, le modèle étant alors un modèle linéaire.

Sur chacun des diagrammes des figures 4A et 4B, une droite 403 représente la fonction $f_{CR}$ déterminée pour le patient, reliant la glycémie $G^r$ du patient à son coefficient de sensibilité à l'insuline CR.

A titre d'exemple, dans le système de la figure 1, la fonction $f_{CR}$ peut être déterminée de façon automatisée par l'unité de traitement et de contrôle 105, lors d'une phase de calibration.

L'unité de contrôle et de traitement 105 peut de plus être configurée pour mettre à jour de façon automatisée, par exemple périodiquement, les paramètres de la fonction $f_{CR}$, pour tenir compte des nouvelles données d'historique enregistrées par le système de régulation au fur et à mesure de son utilisation par le patient.

Dans un mode de réalisation préféré, le nombre $Nb_{ev}$ d'événements pris en compte à chaque mise à jour des paramètres de la fonction $f_{CR}$ reste constant. Autrement dit, à chaque fois qu'un nouvel évènement est pris en compte pour la mise à jour des paramètres de la fonction $f_{CR}$, un évènement antérieur, par exemple l'événement le plus ancien, est exclu du modèle, ce qui permet que le modèle ne se fige pas et puisse évoluer au cours du temps.

Lors des phases de mise à jour du modèle, les mesures qui conduiraient à une modification importante du modèle sont de préférence exclues, de façon que des évènements anormaux ou exceptionnels ne conduisent pas à une instabilité du modèle.

A titre d'exemple, chaque jour, l'unité de contrôle et de traitement 105 peut réaliser une ou plusieurs nouvelles mesures du coefficient de sensibilité à l'insuline réel $CR^r$ du patient, et décider d'incorporer ou non ces mesures au modèle, selon qu'elles répondent ou non à des conditions prédéterminées.

A titre d'exemple, chaque nouvelle mesure du coefficient de sensibilité à l'insuline réel $CR^r$ du patient est incorporée au modèle uniquement si elle répond aux conditions suivantes :

- l'écart entre la nouvelle mesure du coefficient de sensibilité à l'insuline réel $CR^r$ et la valeur du coefficient de sensibilité à l'insuline CR estimé à partir du modèle courant (pour une même valeur de glycémie $G^r$) est inférieur, en valeur absolue, à l'écart type de l'ensemble des valeurs du coefficient $CR^r$ prises en compte dans le modèle courant ; et
- la variation de chacun des paramètres du modèle $f_{CR}$ (les paramètres a et b dans l'exemple susmentionné) liée à l'incorporation de la nouvelle mesure du coefficient $CR^r$ est inférieure à un seuil prédéterminé, par exemple inférieure à un pourcentage P prédéterminé, par exemple compris entre 1 et 20 %, de la valeur du paramètre avant la mise à jour du modèle.

L'unité de traitement et de contrôle 105 peut de plus être configurée pour, après chaque utilisation, par le système de régulation, d'une valeur du coefficient de sensibilité à l'insuline CR estimée à partir du modèle $f_{CR}$, vérifier la pertinence du modèle, et, le cas échéant, corriger le modèle. Plus particulièrement, après chaque utilisation d'une valeur du coefficient de sensibilité à l'insuline CR estimée à partir du modèle $f_{CR}$ pour corriger une hyperglycémie (i.e. pour déterminer un bolus d'insuline à injecter au patient pour corriger l'hyperglycémie), l'unité de traitement et de contrôle 105 peut calculer le coefficient de sensibilité à l'insuline réel $CR^r$ du patient sur une période d'observation passée comprise dans la phase de diminution de la glycémie du patient suivant le pic d'hyperglycémie, puis calculer l'erreur $\varepsilon$ = $CR^r$ - CR entre le coefficient de sensibilité à l'insuline réel et le coefficient de sensibilité à l'insuline estimé. L'unité de traitement et de contrôle 105 peut ensuite multiplier l'erreur $\varepsilon$ par une fonction de coût, par exemple la fonction décrite dans l'article intitulé "Adaptive control in an artificial pancréas for people with type 1 diabetes" de D. Boiroux et al. (J. B. (2017) - Control Engineering Practice, 58, 332-342), puis comparer le résultat de la multiplication à un seuil prédéterminé afin d'estimer si le modèle est pertinent ou non. Si le modèle est jugé non pertinent, une correction peut être apportée au modèle pour une future utilisation.

L'unité de traitement et de contrôle 105 peut en outre être configurée pour estimer la pertinence du modèle pendant un événement, par exemple après une période prédéterminée, par exemple comprise entre 30 et 90 minutes, à compter de l'injection d'un bolus correctif suite à la détection d'une hyperglycémie. L'unité de traitement et de contrôle 105 peut par exemple mesurer le coefficient de sensibilité à l'insuline réel $CR^r$ sur la période considérée, et déterminer le signe de l'erreur $\varepsilon$ entre le coefficient de sensibilité à l'insuline réel $CR^r$ et le coefficient de sensibilité à l'insuline estimé CR. En fonction du signe de l'erreur $\varepsilon$, l'unité de traitement et de contrôle 105 peut commander une injection supplémentaire d'insuline, ou diminuer les doses d'insulines à injecter programmées pour une période à venir, ou encore proposer un resucrage (ingestion de glucose) au patient si le coefficient estimé CR semble trop important.

La figure 5 est une représentation simplifiée du modèle mathématique $f_{CR}$ utilisé dans le système de la figure 1 pour déterminer le coefficient de sensibilité à l'insuline du patient. Sur la figure 5, le modèle est représenté sous la forme d'un bloc de traitement comportant une entrée e3 sur laquelle est appliquée un signal $G^r(t)$ représentatif de la glycémie réelle du patient, mesurée par le capteur 101, à un instant de mesure t, et une sortie s2 fournissant un signal CR(t) représentatif du coefficient de sensibilité à l'insuline du patient à l'instant t.

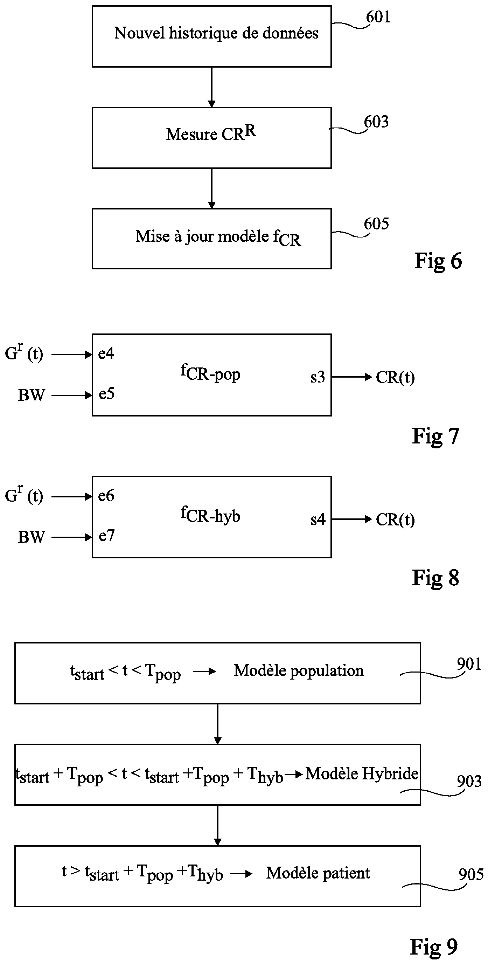

La figure 6 est un diagramme illustrant un exemple d'un procédé pouvant être mis en oeuvre par le système de la figure 1 pour mettre à jour le modèle mathématique $f_{CR}$ pour tenir compte d'un nouvel historique de données du patient.

Le procédé de la figure 6 comprend une étape 601 au cours de laquelle l'unité de traitement et de contrôle 105 acquière et mémorise les données de glycémie réelle $G^r$ mesurée par le capteur 101, les données d'insuline effectivement injectée par le dispositif 103, et les données d'ingestion de glucose par le patient, sur une période d'observation. La période d'observation considérée à l'étape 601 est choisie pour comprendre au moins un évènement permettant une mesure du coefficient de sensibilité réel $CR^r$ du patient.

Le procédé de la figure 6 comprend en outre, après l'étape 601, une étape 603 de calcul du coefficient de sensibilité à l'insuline réel $CR^r$ du patient à partir des données acquises pendant la période d'observation.

Le procédé de la figure 6 comprend de plus, après l'étape 603, une étape 605 de mise à jour du modèle $f_{CR}$ en tenant compte de la nouvelle valeur du coefficient de sensibilité à l'insuline réel $CR^r$ déterminée à l'étape 603.

Comme indiqué précédemment, un historique de données sur une période relativement longue, typiquement de plusieurs semaines à plusieurs mois, est nécessaire pour pouvoir définir un modèle $f_{CR}$ spécifique à un patient donné.

Au début de l'utilisation du système de régulation, avant qu'un historique de données suffisamment important ait été acquis, un modèle générique (i.e. non spécifique au patient) $f_{CR-pop}$, aussi appelé modèle population, peut être utilisé pour calculer en temps réel le coefficient de sensibilité à l'insuline du patient en fonction de sa glycémie. A titre d'exemple, le modèle $f_{CR-pop}$ est déterminé à partir d'une base de données contenant l'historique de glycémie, l'historique d'injection d'insuline, et l'historique de prise de glucose d'un grand nombre de patients, par exemple au moins 20 patients, sur une période de relativement longue, par exemple de plusieurs semaines à plusieurs mois. Pour chaque patient, une pluralité d'événements sont identifiés, et pour chaque événement, une valeur du coefficient de sensibilité à l'insuline $CR^r$ du patient, et une valeur de glycémie $G^r$ correspondante sont déterminées, par exemple de façon identique ou similaire à ce qui a été décrit précédemment en relation avec les figures 4A et 4B. Pour chaque valeur du coefficient de sensibilité à l'insuline $CR^r$ mesurée, le coefficient $CR^r$ est normalisé par le poids du patient. Autrement dit, un coefficient $CRBW^r = CR^r/BW$ est calculé, où BW désigne le poids du patient. Ainsi, pour chaque patient, une pluralité de couples de valeurs $(CRBW^r, G^r)$ sont déterminés. Le modèle $f_{CR-pop}$ est ensuite déterminé par régression à partir de l'ensemble des couples de valeurs $(CRBW^r, G^r)$ déterminés, tous patients confondus. A titre d'exemple, le modèle $f_{CR-pop}$ est déterminé par régression à partir de l'ensemble des couples de valeurs $(CRBW^r, G^r)$ déterminés.

La figure 7 est une représentation simplifiée du modèle mathématique $f_{CR-pop}$ utilisé dans le système de la figure 1 pendant une phase initiale d'utilisation du système, avant qu'un historique de données suffisamment important pour déterminer un modèle spécifique au patient ait été acquis. Sur la figure 7, le modèle est représenté sous la forme d'un bloc de traitement comportant une entrée e4 sur laquelle est appliqué un signal $G^r(t)$ représentatif de la glycémie réelle du patient, mesurée par le capteur 101, à un instant de mesure t, une entrée e5 sur laquelle est appliquée une valeur représentative du poids BW du patient, par exemple en kg, et une sortie s3 fournissant un signal $CR(t) = BW \times f_{CR-pop}(G^r(t))$, représentatif du coefficient de sensibilité à l'insuline du patient à l'instant t.

A poids identiques et à glycémies identiques, deux patients ne présentent toutefois pas nécessairement le même coefficient de sensibilité à l'insuline. Ainsi, il est souhaitable de personnaliser le plus rapidement possible le modèle, dès qu'une quantité de données d'historique suffisante a été acquise.

Dans un mode de réalisation préféré, entre la phase initiale dans laquelle le modèle générique $f_{CR-pop}$ défini ci-dessus est utilisé, et une phase de régime établi dans laquelle un modèle $f_{CR}$ spécifique au patient est utilisé, on prévoit d'utiliser un modèle hybride $f_{CR-hyb}$, défini comme suit.

Le modèle $f_{CR-hyb}$ est défini après une certaine période $T_{pop}$ d'utilisation du système de régulation sur la base du modèle population $f_{CR-pop}$, par exemple une période de 1 à 7 jours, par exemple une période de l'ordre de 3 jours. A l'issue de la période $T_{pop}$, l'unité de traitement et de contrôle 105 calcule un coefficient k spécifique au patient, selon la formule suivante :

$$k = \frac{TDD_{moy}}{a1 * BW}$$

où BW désigne le poids du patient, $TDD_{moy}$ désigne la dose d'insuline journalière moyenne injectée au patient sur la période $T_{pop}$, et a1 est un coefficient constant, par exemple compris entre 0,5 et 0,9. Le modèle hybride $f_{CR-hyb}$ est alors défini comme suit :

$$f_{CR-hyb} = k * f_{CR-pop}$$

A titre de variante, le coefficient k est calculé selon la formule suivante :

$$k = \frac{TDD_{basal}}{a2 * BW}$$

où $TDD_{basal}$ désigne la dose d'insuline journalière de base (i.e. en l'absence de tout bolus de correction) injectée au patient sur la période $T_{pop}$, et a2 est un coefficient constant, par exemple compris entre 0,2 et 0,5.

La figure 8 est une représentation simplifiée du modèle mathématique $f_{CR-hyb}$ utilisé dans le système de la figure 1 pendant une phase intermédiaire $T_{hyb}$ d'utilisation du système, après la phase $T_{pop}$ et avant qu'un historique de données suffisamment important pour déterminer un modèle spécifique au patient ait été acquis. Sur la figure 8, le modèle est représenté sous la forme d'un bloc de traitement comportant une entrée e6 sur laquelle est appliquée un signal $G^r(t)$ représentatif de la glycémie réelle du patient, mesurée par le capteur 101, à un instant de mesure t, une entrée e7 sur laquelle est appliquée une valeur représentative du poids BW du patient, par exemple en kg, et une sortie s4 fournissant un signal

$$CR(t) = BW \times f_{CR-hyb}(G^r(t)) = k \times BW \times f_{CR-pop}(G^r(t))$$

représentatif du coefficient de sensibilité à l'insuline du patient à l'instant t.

La figure 9 est un diagramme illustrant de façon schématique un exemple d'un procédé de contrôle de la glycémie d'un patient pouvant être mis en oeuvre par le système de la figure 1. On considère ici un procédé dans lequel le coefficient de sensibilité à l'insuline CR du patient est successivement estimé une pluralité de fois à partir d'un modèle mathématique, en tenant compte, à chaque estimation, d'une unique valeur de glycémie mesurée par le capteur de glycémie 101 du système.

Lors d'une phase initiale 901 de durée $T_{pop}$, allant d'un instant initial $t_{start}$ de début d'utilisation du système de régulation par le patient à l'instant $t_{start}+T_{pop}$, l'unité de traitement et de contrôle 105 utilise le modèle population $f_{CR-pop}$ pour estimer le coefficient de sensibilité à l'insuline CR du patient. A la fin de la phase 901, l'unité de traitement et de contrôle 105 détermine un modèle partiellement personnalisé $f_{CR-hyb}$ à partir des données acquises pendant la phase 901.

Lors d'une phase intermédiaire 903 de durée $T_{hyb}$, allant de l'instant $t_{start}+T_{pop}$ à l'instant $t_{start}+T_{pop}+T_{hyb}$, l'unité de traitement et de contrôle 105 utilise le modèle hybride $f_{CR-hyb}$ pour estimer le coefficient de sensibilité à l'insuline CR du patient. A la fin de la phase 903, l'unité de traitement et de contrôle 105 détermine un modèle spécifique au patient $f_{CR}$ à partir des données acquises pendant les phases 901 et 903.

Lors d'une phase de régime établi 905 postérieure à l'instant $t_0+T_{pop}+T_{hyb}$, l'unité de traitement et de contrôle 105 utilise le modèle patient $f_{CR}$ pour calculer le coefficient de sensibilité à l'insuline CR du patient. L'unité de traitement et de contrôle 105 peut en outre régulièrement mettre à jour le modèle patient $f_{CR}$ pour tenir compte des nouvelles données acquises pour le patient.

Des modes de réalisation particuliers ont été décrits. Diverses variantes et modifications apparaîtront à l'homme de l'art. En particulier, les modes de réalisation décrits ne se limitent pas à l'exemple particulier de système de régulation de glycémie décrit en relation avec les figures 1 à 3, à savoir un système à commande prédictive utilisant un modèle mathématique pour prédire l'évolution future de la glycémie du patient et ajuster en conséquence les doses d'insuline à administrer au patient. Plus généralement, la méthode proposée d'estimation en temps réel du coefficient de sensibilité à l'insuline CR du patient, à partir d'une unique valeur de glycémie mesurée sur le patient, peut être mise en oeuvre dans tout système de régulation de glycémie pouvant tirer profit d'une estimation in-situ et en temps réel du coefficient de sensibilité à l'insuline du patient.

**Revendications**

1. Système automatisé de contrôle de la glycémie d'un patient, comportant un capteur de glycémie (101) et une unité de traitement et de contrôle (105), **caractérisé en ce que** l'unité de traitement et de contrôle (105) est configurée pour calculer, à partir d'un premier modèle mathématique $f_{CR}$ spécifique au patient et en tenant compte d'une unique valeur de glycémie $G^r$ mesurée par le capteur, un coefficient CR représentatif de la sensibilité à l'insuline du patient.

2. Système selon la revendication 1, comportant en outre un dispositif d'injection d'insuline (103), dans lequel l'unité de traitement et de contrôle (105) est configurée pour commander le dispositif d'injection d'insuline en tenant compte du coefficient CR.

3. Système selon la revendication 2, dans lequel l'unité de traitement et de contrôle (105) est configurée pour prédire, à partir d'un deuxième modèle mathématique (201), l'évolution future de la glycémie du patient sur une période de prédiction, et à commander le dispositif d'injection d'insuline (103) en tenant compte de cette prédiction.

4. Système selon l'une quelconque des revendications 1 à 3, dans lequel le premier modèle mathématique est une fonction d'équation

$$CR = f_{CR}(G^r) = a \times G^{r\,b} + c$$

où a, b et c sont des paramètres spécifiques au patient.

5. Système selon l'une quelconque des revendications 1 à 4, dans lequel l'unité de traitement et de contrôle (105) est configurée pour mettre en oeuvre une étape de calibration automatique du premier modèle $f_{CR}$ en tenant compte d'un historique de glycémie mesurée par le capteur (101), d'un historique d'insuline injectée au patient, et d'un historique d'ingestion de glucose par le patient sur une période d'observation passée.

6. Système selon la revendication 5, dans lequel l'unité de traitement et de contrôle (105) est configurée pour, lors de l'étape de calibration automatique, mesurer une pluralité de valeurs du coefficient de sensibilité à l'insuline réel $CR^r$ du patient lors d'une pluralité d'événements de mesure compris dans la période d'observation passée.

7. Système selon la revendication 6, dans lequel chaque événement de mesure correspond à une plage temporelle continue allant d'un instant initial $t_{init}$ à un instant final $t_{final}$, répondant aux critères suivants :

- l'instant $t_{init}$ se situe dans une phase d'hyperglycémie, c'est-à-dire une phase dans laquelle la glycémie du patient est supérieure à un seuil prédéterminé ;
- un bolus de correction, c'est-à-dire une dose d'insuline, a été administré au patient après le début de la phase d'hyperglycémie et avant l'instant $t_{init}$, en vue de limiter la durée de la phase d'hyperglycémie ;
- la glycémie du patient décroit de façon continue entre l'instant initial $t_{init}$ et l'instant final $t_{final}$ ;
- aucune ingestion de glucose par le patient n'a été effectuée entre l'instant $t_{init} - T_j$ et l'instant $t_{final}$, où $T_j$ est une durée de jeun prédéterminée.

8. Système selon la revendication 6 ou 7, dans lequel l'unité de traitement et de contrôle (105) est configurée pour, lors de l'étape de calibration automatique, déterminer le premier modèle mathématique $f_{CR}$ par régression à partir de ladite pluralité de valeurs du coefficient de sensibilité à l'insuline réel $CR^r$.

9. Système selon l'une quelconque des revendications 5 à 8, dans lequel l'unité de traitement et de contrôle (105) est configurée pour, lors d'une phase initiale $T_{pop}$ d'utilisation du système précédant l'étape de calibration automatique du premier modèle, utiliser un modèle mathématique générique $f_{CR-pop}$ non personnalisé pour calculer le coefficient CR.

10. Système selon la revendication 9, dans lequel l'unité de traitement et de contrôle (105) est configurée pour, lors d'une phase intermédiaire $T_{hyb}$ d'utilisation du système, postérieure à la phase initiale $T_{pop}$ et antérieure à l'étape de calibration automatique du premier modèle, utiliser un modèle mathématique partiellement personnalisé $f_{CR-hyb}$ pour calculer le coefficient CR.

11. Système selon la revendication 10, dans lequel le modèle mathématique partiellement personnalisé est défini par l'équation :

$$f_{CR-hyb}(G^r) = k \times f_{CR-pop}(G^r)$$

où k est un coefficient spécifique au patient défini selon la formule suivante :

$$k = \frac{TDD_{moy}}{a1 \times BW}$$

où BW désigne le poids du patient, $TDD_{moy}$ désigne la dose d'insuline journalière moyenne injectée au patient sur la période $T_{pop}$, et a1 est un coefficient constant compris entre 0,5 et 0,9.

12. Système selon l'une quelconque des revendications 5 à 11, dans lequel l'unité de traitement et de contrôle (105) est configurée pour, après l'étape de calibration automatique du premier modèle $f_{CR}$, mettre en oeuvre une pluralité d'étapes successives de re-calibration du premier modèle pour tenir compte de nouvelles données de glycémie mesurée par le capteur (101), d'insuline injectée au patient, et d'ingestion de glucose par le patient.

**Patentansprüche**

1. Automatisiertes System zum Steuern des Blutzuckers eines Patienten, welches einen Blutzuckersensor (101) und eine Verarbeitungs- und Steuereinheit (105) aufweist, **dadurch gekennzeichnet, dass** die Verarbeitungs- und Steuereinheit (105) konfiguriert ist, um einen Faktor CR zu berechnen, der repräsentativ ist für die Insulinsensitivität bzw. Insulinempfindlichkeit des Patienten, und zwar aus einem ersten mathematischen Modell $f_{CR}$ , das für den Patienten spezifisch ist, und unter Berücksichtigung eines einzelnen Blutzuckerwertes $G^r$, der von dem Sensor gemessen wird.

2. System nach Anspruch 1, welches ferner eine Insulininjektionsvorrichtung (103) aufweist, wobei die Verarbeitungs- und Steuereinheit (105) konfiguriert ist, um die Insulininjektionsvorrichtung zu steuern, und zwar unter Berücksichtigung des Faktors CR.

3. System nach Anspruch 2, wobei die Verarbeitungs- und Steuereinheit (105) konfiguriert ist, um aus einem zweiten mathematischen Modell (201) die zukünftige Entwicklung des Blutzuckers des Patienten über einen Vorhersagezeitraum vorherzusagen, und um die Insulininjektionsvorrichtung (103) unter Berücksichtigung der Vorhersage zu steuern.

4. System nach einem der Ansprüche 1 bis 3, wobei das erste mathematische Modell eine Funktion der folgenden Gleichung ist

$$CR = f_{CR}(G^r) = a \times G^{rb} + c \; ,$$

wobei a, b und c Parameter sind, die für den Patienten spezifisch sind.

5. System nach einem der Ansprüche 1 bis 4, wobei die Verarbeitungs- und Steuereinheit (105) konfiguriert ist, um einen Schritt zur automatischen Kalibrierung des ersten Modells $f_{CR}$ zu implementieren, wobei Folgendes berücksichtigt wird: ein Verlauf des Blutzuckers, der von dem Sensor (101) gemessen wird, ein Verlauf des Insulins, welches in den Patienten injiziert wird, und ein Verlauf der Kohlenhydrataufnahme durch den Patienten über einen vergangenen Beobachtungszeitraum.

6. System nach Anspruch 5, wobei die Verarbeitungs- und Steuereinheit (105) konfiguriert ist, um während des automatischen Kalibrierungsschrittes eine Vielzahl von Werten des tatsächlichen Insulinempfindlichkeitsfaktors $CR^r$ des Patienten zu messen, und zwar während einer Vielzahl von Messereignissen, die im vergangenen Beobachtungszeitraum enthalten sind.

7. System nach Anspruch 6, wobei jedes Messereignis einem kontinuierlichen bzw. durchgehenden Zeitbereich von einem Anfangszeitpunkt $t_{init}$ bis zu einem Endzeitpunkt $t_{final}$ entspricht, welcher die folgenden Kriterien erfüllt:

der Zeitpunkt $t_{init}$ befindet sich in einer Hyperglykämiephase, d.h. in einer Phase, in der der Blutzucker des Patienten höher als ein vorbestimmter Schwellenwert ist ;
ein Korrekturbolus, das heißt eine zusätzliche Dosis Insulin wurde dem Patienten nach dem Beginn der Hyperglykämiephase und vor dem Zeitpunkt $t_{init}$ verabreicht, um die Dauer der Hyperglykämiephase zu begrenzen;
der Blutzucker des Patienten nimmt zwischen dem Anfangszeitpunkt tinit und dem Endzeitpunkt $t_{final}$ kontinuierlich ab;
zwischen dem Zeitpunkt $t_{init}$ - Tj und dem Zeitpunkt $t_{final}$, fand keine Kohlenhydrataufnahme durch den Patienten statt, wobei Tj eine vorbestimmte Fastenzeitdauer ist.

8. System nach Anspruch 6 oder 7, wobei die Verarbeitungs- und Steuereinheit (105) konfiguriert ist, um während des automatischen Kalibrierungsschritts das erste mathematische Modell $f_{CR}$ durch Regression aus der Vielzahl von Werten des tatsächlichen Insulinempfindlichkeitsfaktors $CR^r$ zu bestimmen.

9. System nach einem der Ansprüche 5 bis 8, wobei die Verarbeitungs- und Steuereinheit (105) konfiguriert ist, um während einer Anfangsphase $T_{pop}$ der Nutzung des Systems, die dem Schritt der automatischen Kalibrierung des ersten Modells vorangeht, ein nicht personalisiertes generisches mathematisches Modell $f_{CR-pop}$ zu verwenden, um den Faktor CR zu berechnen.

10. System nach Anspruch 9, wobei die Verarbeitungs- und Steuereinheit (105) konfiguriert ist, um während einer Zwischenphase $T_{hyb}$ der Nutzung des Systems, und zwar anschließend an die Anfangsphase $T_{pop}$ und vor dem Schritt der automatischen Kalibrierung des ersten Modells, ein teilweise personalisiertes mathematisches Modell $f_{CR-hyb}$ zu verwenden, um den Faktor CR zu berechnen.

11. System nach Anspruch 10, wobei das teilweise personalisierte mathematische Modell durch folgende Gleichung definiert ist:

$$f_{CR-hyb}(G^r) = k \times f_{CR-pop}(G^r)$$

wobei k ein für den Patienten spezifischer Faktor ist, der gemäß der folgenden Formel definiert wird:

$$k = \frac{TDD_{moy}}{a1 \times BW}$$

wobei BW das Gewicht des Patienten bezeichnet, $TDD_{moy}$ die durchschnittliche tägliche Insulindosis bezeichnet, die dem Patienten über die Periode $T_{pop}$ injiziert wird, und a1 ein konstanter Faktor im Bereich von 0,5 bis 0,9 ist.

12. System nach einem der Ansprüche 5 bis 11, wobei die Verarbeitungs- und Steuereinheit (105) konfiguriert ist, um nach dem Schritt der automatischen Kalibrierung des ersten Modells $f_{CR}$ eine Vielzahl von aufeinanderfolgenden Schritten zur Rekalibrierung bzw. Neukalibrierung des ersten Modells zu implementieren, um neue Daten des Blutzuckers, die von dem Sensor (101) gemessen werden, des Insulins, welches dem Patienten injiziert wird und die Kohlenhydrataufnahme durch den Patienten zu berücksichtigen.

**Claims**

1. An automated system for controlling a patient's blood glucose, comprising a blood glucose sensor (101) and a processing and control unit (105), **characterized in that** the processing and control unit (105) is configured to calculate, from a first mathematical model $f_{CR}$ specific to the patient and taking into account a single blood glucose value $G^r$ measured by the sensor, a factor CR representative of the patient's insulin sensitivity.

2. The system according to claim 1, further comprising an insulin injection device (103), wherein the processing and control unit (105) is configured to control the insulin injection device by taking into account factor CR.

3. The system according to claim 2, wherein the processing and control unit (105) is configured to predict, from a second mathematical model (201), the future trend of the patient's blood glucose over a prediction period, and to control the insulin injection device (103) by taking the prediction into account.

4. The system according to any of claims 1 to 3, wherein the first mathematical model is a function of equation

$$CR = f_{CR}(G^r) = a \times G^{rb} + c$$

where a, b, and c are parameters specific to the patient.

5. The system according to any of claims 1 to 4, wherein the processing and control unit (105) is configured to implement a step of automatic calibration of first model $f_{CR}$ by taking into account a history of the blood glucose measured by the sensor (101), a history of insulin injected to the patient, and a history of carbohydrate ingestion by the patient over a past observation period.

6. The system according to claim 5, wherein the processing and control unit (105) is configured to, during the automatic calibration step, measure a plurality of values of the patient's real insulin sensitivity factor $CR^r$ during a plurality of measurement events contained in the past observation period.

7. The system according to claim 6, wherein each measurement event corresponds to a continuous time range from an initial time $t_{init}$ to a final time $t_{final}$, complying with the following criteria:

   - time $t_{init}$ is in a hyperglycemia phase, that is, a phase where the patient's blood glucose is greater than a predetermined threshold;
   - a correction bolus, that is, an additional insulin dose has been delivered to the patient after the beginning of the hyperglycemia phase and before time $t_{init}$, to limit the duration of the hyperglycemia phase;
   - the patient's blood glucose continuously decreases between initial time $t_{init}$ and final time $t_{final}$;
   - no carbohydrate ingestion by the patient has occurred between time $t_{init}$ - Tj and time $t_{final}$, where $T_j$ is a predetermined fasting duration.

8. The system according to claim 6 or 7, wherein the processing and control unit (105) is configured to, during the automatic calibration step, determine the first mathematical model $f_{CR}$ by regression from said plurality of values of the real insulin sensitivity factor $CR^r$.

9. The system according to any of claims 5 to 8, wherein the processing and control unit (105) is configured to, during an initial phase $T_{pop}$ of use of the system preceding the step of automatic calibration of the first model, use a non-personalized generic mathematical model $f_{CR-pop}$ to calculate factor CR.

10. The system according to claim 9, wherein the processing and control unit (105) is configured to, during an intermediate phase $T_{hyb}$ of use of the system, subsequent to the initial phase $T_{pop}$ and preceding the step of automatic calibration of the first model, use a partially personalized mathematical model $f_{CR-hyb}$ to calculate factor CR.

11. The system according to claim 10, wherein the partially personalized mathematical model is defined by equation:

$$f_{CR-hyb}(G^r) = k \times f_{CR-pop}(G^r)$$

where k is a factor specific to the patient defined according to the following formula:

$$k = \frac{TDD_{moy}}{a1 \times BW}$$

where BW designates the patient's weight, $TDD_{moy}$ designates the average daily insulin dose injected to the patient over period $T_{pop}$, and a1 is a constant factor in the range from 0.5 to 0.9.

12. The system according to any of claims 5 to 11, wherein the processing and control unit (105) is configured to, after the step of automatic calibration of first model $f_{CR}$, implement a plurality of successive steps of re-calibration of the first model to take into account new data of blood glucose measured by the sensor (101), of insulin injected to the patient, and of carbohydrate ingestion by the patient.

Fig 1

Fig 2

Fig 3

CR, CRʳ (UI/g/ℓ)

Fig 4A

CR, CRʳ (UI/g/ℓ)

Fig 4B

Fig 5

Nouvel historique de données — 601

Mesure $CR^R$ — 603

Mise à jour modèle $f_{CR}$ — 605

**Fig 6**

$G^r(t)$ → e4

BW → e5

$f_{CR-pop}$

s3 → CR(t)

**Fig 7**

$G^r(t)$ → e6

BW → e7

$f_{CR-hyb}$

s4 → CR(t)

**Fig 8**

$t_{start} < t < T_{pop}$ → Modèle population — 901

$t_{start} + T_{pop} < t < t_{start} + T_{pop} + T_{hyb}$ → Modèle Hybride — 903

$t > t_{start} + T_{pop} + T_{hyb}$ → Modèle patient — 905

**Fig 9**

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- FR 1800492
- US 2014276556 A
- FR 1658881
- FR 1658882
- FR 1756960
- US 20100198520 A
- US 20130211220 A
- WO 2017040927 A

**Littérature non-brevet citée dans la description**

- **ROMAN HOVORKA et al.** *Physiol Meas.,* 2004, vol. 25, 905-920
- **ROMAN HOVORKA et al.** *Am J Physiol Endocrinol Metab,* 2002, vol. 282, E992-E1007
- **D. BOIROUX et al.** Adaptive control in an artificial pancréas for people with type 1 diabetes. *Control Engineering Practice,* 2017, vol. 58, 332-342